(19) Europäisches Patentamt
European Patent Office
Office européen des brevets

(11) EP 2 144 593 B1

(12) # EUROPÄISCHE PATENTSCHRIFT

(45) Veröffentlichungstag und Bekanntmachung des Hinweises auf die Patenterteilung:
**27.09.2017 Patentblatt 2017/39**

(21) Anmeldenummer: **08749638.6**

(22) Anmeldetag: **22.04.2008**

(51) Int Cl.:
***A61K 8/39*** *(2006.01)* ***A61K 8/60*** *(2006.01)*
***A61Q 19/10*** *(2006.01)*

(86) Internationale Anmeldenummer:
**PCT/EP2008/054834**

(87) Internationale Veröffentlichungsnummer:
**WO 2008/138708 (20.11.2008 Gazette 2008/47)**

(54) **HAUT- UND HÄNDEREINIGUNGSMITTEL MIT HYDROPHILEN EMOLLIENTS**
SKIN CLEANSER/HAND CLEANERS COMPRISING HYDROPHILIC EMOLLIENTS
PRODUITS NETTOYANTS POUR LA PEAU ET LES MAINS À ÉMOLLIENTS HYDROPHILES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **11.05.2007 DE 102007022693**

(43) Veröffentlichungstag der Anmeldung:
**20.01.2010 Patentblatt 2010/03**

(60) Teilanmeldung:
**12155687.2 / 2 455 062**

(73) Patentinhaber: **Deb IP Limited**
**Denby, Derbyshire DE5 8JZ (GB)**

(72) Erfinder:
- **ALLEF, Petra**
  **47807 Krefeld (DE)**
- **KLOTZBACH, Volker**
  **47669 Wachtendonk (DE)**
- **VEEGER, Marcel**
  **47574 Goch (DE)**

(74) Vertreter: **Thurston, Joanna et al**
**Withers & Rogers LLP**
**4 More London Riverside**
**London SE1 2AU (GB)**

(56) Entgegenhaltungen:
**DE-A1-102004 025 287**
**US-A- 5 658 577**
**US-A- 5 888 951**
**US-A1- 2006 165 635**
**US-A1- 2007 072 780**
**US-B1- 6 432 430**

EP 2 144 593 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft Haut- und Händereinigungsmittel mit hydrophilen Emollients mit verbesserter dermatologischer Verträglichkeit und die Verwendung der Mittel zur Entfernung von extremen Verschmutzungen.

Haut- und Handreinigungsmittel werden in der Industrie als sogenannte Grobhandreiniger umfangreich eingesetzt, insbesondere dort, wo hartnäckige Verschmutzungen auftreten, die durch Lacke, Fette, Öle, Schmierstoffe, Metallstäube, Graphit, Ruß u.a. hervorgerufen werden.

Je häufiger derartige Produkte im industriellen Bereich (bis zu 6mal und öfters am Tag) auf der Haut angewendet werden, desto deutlicher treten die nachteiligen Wirkungen der in diesen Grobhandreinigern enthaltenen Abrasiva, Tenside bzw. Tensidgemische und insbesondere auch Lösungsmittel, wie z.B. aliphatische Kohlenwasserstoffe, Terpene, Carbonsäureester vom Typ Dimethyladipat, Dimethylglutarat, Dimethylsuccinat (DBE) und Di-n-butyladipat bzw. Di-isopropyladipat, die in der DE 43 35933 A1 beschrieben worden sind, in den Vordergrund, nämlich die Entfettung und Austrocknung der Haut durch die Zerstörung des Hydro-Lipid-Mantels der Haut. Es kommt dann zu verstärkter Resorption toxischer und allergener Stoffe oder zum Befall von Mikroorganismen oder als Folge zu toxischen oder allergenen Hautreaktionen.

Zur Vermeidung der vorgenannten Hautproblematik sind in der Patentliteratur eine Vielzahl von Formulierungen für Grobhandreiniger vorgeschlagen worden. So beschreibt beispielsweise die WO 99/06021 wasserhaltige sogenannte "waterless handc-leaner"-Formulierungen, die einen Wassergehalt von 75 bis 99 Gew.-%, bis zu 25 Gew.-% eines Fettsäureesters oder Mischungen solcher Ester sowie mindestens ein Tensid aufweisen. Solche Formulierungen stellen gegenüber den zuvor erwähnten organische Lösungsmittel enthaltenden Produkten, insbesondere in toxikologischer Hinsicht gesehen eine deutliche Verbesserung dar. Nichtsdestoweniger ging die verbesserte Hautverträglichkeit sowie der verbesserte Schutz vor Hautaustrocknung ein-her mit einem Verlust an Reinigungswirkung im Vergleich zu lösemittelhaltigen Grobhandreiniger.

Die DE 197 48 921 A1 betrifft wasserhaltige flüssige, pasten- oder cremeförmige Handreinigungsmittel, insbesondere Grobhandreinigungsmittel mit einem Reibemittel, die einen Gehalt von 10 bis 30 Gew.-% wenigstens eines pflanzlichen Öles, gewählt aus der Gruppe der Triglyceride, gesättigten und/oder ungesättigten Fettsäuren, von 10 bis 30 Gew.-% wenigstens eines Tensids aus der Gruppe Fettalkoholethoxylate, Fettalko-holethersulfate und/oder Rizinusölsulfonate sowie 10 bis 65 Gew.-% Wasser, jeweils bezogen auf die Zusammensetzung der Reinigungsmittel. Als Grobhandreiniger enthalten diese Mittel in der Zusammensetzung 1 bis 30 Gew.-% wenigstens ein Reibmittel. Die dort beschriebenen Grobhandreiniger sind aus dermatologischer Sicht sehr gut hautverträglich und weisen auch bei mehrmaliger täglicher Anwendung nur eine geringe Hautaustrocknung auf.

Aus der EP 1 152 051 A2 sind hautmilde Handwaschpasten bekannt, wobei Fettsäurealkylester in Kombination mit Emulgatoren, bevorzugt Fettalkoholethoxylaten eingesetzt werden. Als Rückfettungsmittel werden zusätzlich Partialglyceride eingesetzt.

Obwohl die Rückfettungsmittel in dieser Druckschrift umfangreich beschrieben werden, beziehen sich die Beispiele nur auf Emulsionen mit 20 bis 30 Gew.-% Fettsäureestern und zusätzlich 10 Gew.-% eines Gemisches aus Cocoglycoside und Glyceryl oleate (Lamesoft PO65). Es ist anzunehmen, dass die rückfettende Wirkung ähnlich wie bei den in der DE 197 48 921 C2 beschriebenen Handreinigungsmitteln auf dem Gehalt an Fettsäureestern beruhen. Dennoch führen auch die Handwaschpasten gemäß EP 1 152 051 A2 bei häufiger Anwendung zu Hautaustrocknung.

Weiterhin beschreibt auch DE 4009534 A1 ein flüssiges Handreinigungsmittel und offenbart hierzu ein Reinigungsmittel mit einer Kombination aus hydriertem Rizinusöl und einem organischen Lösungsmittel gemäß der Formel R-CO-$(OCH2CH2)_n$-OX, wobei R ein Alkylrest mit 1 bis 3 C-Atomen, n eine Zahl zwischen 1 und 4 und X ein Alkylrest mit 1 bis 5 C-Atomen bedeutet.

Die DE 199 16 036 A1 beschreibt Handwaschpasten, enthaltend anionische Tenside, Reibkörper und Partialglyceride, insbesondere Ölsäuremonoglyceride, sowie die Verwendung derartiger Partialglyceride zur Verbesserung des Hautgefühls. In den Beispielen wird jedoch ausschließlich ein Gemisch aus Cocoglycoside und Glyceryl oleate (Lamesoft PO65) verwendet. Obgleich die in dieser Offenlegungsschrift beschriebenen Handwaschpasten zu einer subjektiven Verbesserung des Hautgefühls führen sollen, werden keine experimentellen Daten hierfür angegeben, die eine solche Verbesserung des Hautzustandes belegen könnten.

Nichtsdestoweniger wird auch eine solch subjektive Verbesserung des Hautgefühls bei häufigem Gebrauch solcher Grobhandreiniger von den Anwendern als hautaustrocknend empfunden, trotz der Tatsache, dass die Grobhandreiniger gemäß DE 197 48 921 A1, die bis zu 30 Gew.-% pflanzlicher Öle, vorzugsweise Rüböl als Rückfettungsmittel bzw. hydrophobes Emollient enthalten. Gleiches gilt für die EP 1 152 051 A1, die bis zu 40 Gew.-% Fettsäurealkylester als hydrophobes Emollient enthalten. Darüber hinaus ist hinsichtlich der in der DE 199 16 036 A1 beschriebenen Formulierungen, die Partialglyceride enthalten, festzustellen, das diese bei häufiger Anwendung zu einer Verstärkung der Hautaustrocknung führen.

Somit besteht weiterhin ein Bedarf an milden Haut- bzw. Handreinigungsmittel, die eine sehr gute Reinigungswirkung auch bei hartnäckigsten Hautverschmutzungen gewähr-leisten bei gleichzeitiger sehr guter Hautverträglichkeit.

Insbesondere sollen diese Grobhandreiniger bereits während des Waschens ein sehr gutes Hautgefühl erzeugen, wobei diese sehr gute Hautanmutung aufgrund der geringen Hautaustrocknung auch nach dem Waschen für den Anwender noch einige Zeit wahrnehmbar bleibt, und so die Akzeptanz der Anwender hinsichtlich der Verwendung von Grobhandreinigern erhöht wird.

Aufgabe war es daher, solche Haut- und Handreinigungsmittel, insbesondere Grobhandreinigungsmittel bereitzustellen. Diese Aufgabe wurde überraschend gelöst durch ein Haut- und Handreinigungsmittel, die einen Gehalt von mindestens 0,1 Gew.-% von wenigstens einem hydrophilen Emollient mit einem HLB- Wert von ≥ 10 aufweisen, wobei Polyolester als hydrophile Emollients besonders bevorzugt sind.

Erfindungswesentlich für diese Haut- und Handreinigungsmittel ist hierbei, dass die hydrophilen Emollients einen HLB- Wert von ≥ 10 aufweisen. Der HLB-Wert ist ein Maß für die Wasser- bzw. Öl-Löslichkeit von vorwiegend nichtionischen Tensiden und Emulgatoren beispielsweise in kosmetischen Produkten und beschreibt somit den hydrophilen Teil und den lipophilen Anteil einer chemischen Verbindung. So entspricht ein HLB-Wert von 0 einer rein lipophilen Verbindung. Demgegenüber hat eine chemische Verbindung mit einem HLB-Wert von 20 nur hydrophile Anteile. So werden Wasser-in-Öl-Emulsionen HLB-Werte zwischen 3 bis 8 zugeordnet, während Öl-inWasser-Emulsionen HLB-Werte zwischen 8 bis 18 sowie Waschmittel HLB-Werte zwischen 13 bis 15 haben (siehe auch RÖMPP - Chemielexikon, 10. Auflage, Seite 1764, Stichworte: HLB-System, HLB-Wert - sowie dort zitierte Literatur).

Für die hier vorliegende Erfindung werden Emollients mit einem HLB-Wert zwischen 1 bis 8 als hydrophob, mit 8 bis 10 als neutral bzw. ≥ 10 als hydrophil bezeichnet.

Die erfindungsgemäßen hydrophilen Emollients weisen im Gegensatz zu den bisher im Stand der Technik verwendeten überwiegend lipophilen Emollients einen HLB von ≥10, bevorzugt ≥12 auf. So hat das in DE 199 16 036 A1 beschriebene Gemisch aus Cocoglycoside und Glyceryl oleate (Lamesoft PO65) einen HLB-Wert von 9 bzw. Fettsäureester haben einen noch deutlich tieferen HLB-Wert. Es war daher völlig überraschend, obwohl die erfindungsgemäßen hydrophilen Emollients deutlich hydrophiler und somit auch deutlich besser wasserlöslich und damit leichter abzuwaschen sind als die im vorgenannten Stand der Technik verwendeten Emollients, d.h. es also nicht zu einer Rückfettung im eigentlichen Sinne kommt, dass das Hautgefühl nach häufiger Anwendung von den Anwendern subjektiv nicht nur als weniger trocken beschrieben wird, sondern der verbesserte Hautzustand kann auch objektiv mittels Corneometer-Messungen quantifiziert werden wie im experimentellen Teil gezeigt werden wird.

Erfindungsgemäß bevorzugt weisen die erfindungsgemäßen Haut- und Handreinigungsmittel einen Gehalt, jeweils bezogen auf die Gesamtzusammensetzung des Reinigungsmittels, von den Komponenten

a.) mindestens 0,1 Gew.-% von wenigstens einem Polyolester als hydrophilen Emollient mit einem HLB-Wert von ≥ 10
b.) 2 bis 40 Gew.-% wenigstens ein Tensid ausgewählt aus der Gruppe der Fettalkoholethoxylate, Fettalkoholethersulfate und Salze sulfierter und/oder sulfonierter Fettsäuren, gekennzeichnet dadurch dass das Mittel wenigstens ein Fettalkoholethoxylat enthält,
c.) 30 bis 90 Gew.-% Wasser,
d.) 0 bis 30 Gew.-% eines oder mehrerer Reinigungsverstärker ausgewählt aus der Gruppe der Polyole, Polyether, Polyphosphate und Phosphate,
e.) 5 bis 30 Gew.-% eines oder mehrerer Abrasiva,
f.) 0 bis 1,0 Gew.-% propoxylierte Fettalkohole,
g.) gegebenenfalls ein oder mehrere viskositätsbildende Mittel,
h.) gegebenenfalls weitere kosmetische Hilfs-, Zusatz- und/oder Wirkstoffe,

wobei die Summe der Komponenten a.) bis h.) 100 Gew. % ergibt, auf.

Bei den als Komponente a.) einzusetzenden Polyolestern, die in den erfindungsgemäßen Haut- und Handreinigungsmittel als hydrophiles Emollient anzusehen sind, handelt es sich beispielsweise um Partialglyceride, insbesondere um Polyglycerylpartialester. Erfindungsgemäß bevorzugt sind Polyglycerylpartialester der allgemeinen Formel

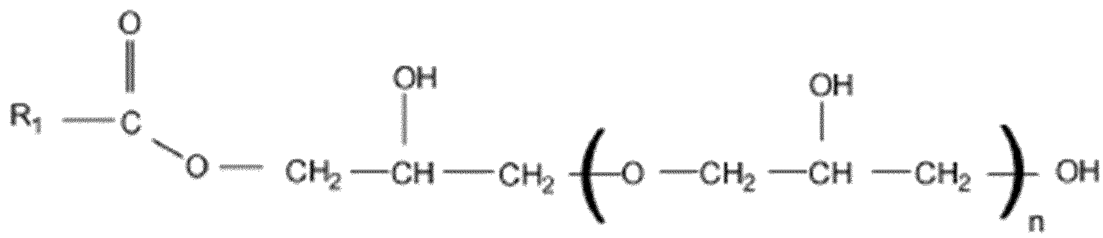

wobei

$R_1$ = linearer, verzweigter oder cyclischer, gesättigter oder ungesättigter Alkyl- bzw. Alkenylrest mit 6 bis 18 Kohlenstoffatomen und n = ganze Zahl von 1 bis 9 sind.

Der lineare, verzweigte oder cyclische, gesättigte oder ungesättigte Alkyl- bzw. Alkenylrest hat insbesondere 6 bis 16 und besonders bevorzugt 8 bis 12 Kohlenstoffatomen, wobei bevorzugt n = 1 bis 5, insbesondere n = 1 bis 3 ist. Weiterhin können auch Polyglycerinester mit anderer Struktur, z.B. auf Basis von 1,2-oder 1,3 verknüpften Polyglycerinen, sowie Polyglycerinether verwendet werden.

Bei den erfindungsgemäß besonders bevorzugten Polyglycerylpartialestern bzw. Polyglycerinfettsäureester handelt es sich beispielsweise um Polyglyceryl-3-caprat bzw. Polyglyceryl-4-caprat, die von der Fa. Degussa unter der Bezeichnung TEGOSOFT® PC31 sowie TEGOSOFT® PC41 erhältlich sind.

Polyolester im Sinne der vorliegenden Erfindung sind desweiteren Polyethylenglycolester, wie z.B. PEG-7 Glyceryl Cocoate, das von der Firma Croda Chemicals Europe Ltd. unter der Bezeichnung Glycerox HE erhältlich ist.

Als hydrophile Emollients enthält die Komponente a.) in den erfindungsgemäßen Haut- und Handreinigungsmittel auch Polysaccharidester mit einem HLB $\geq$10, bevorzugt $\geq$12 enthalten. Bevorzugt werden Sucroseester wie sie beispielsweise von der Fa. Degussa GmbH unter den Bezeichnungen TEGOSOFT® LSE 65 K oder TEGOSOFT® LSE 65 K Soft bezogen werden können.

Der Anteil der Komponente a.) in den erfindungsgemäß Haut- und Handreinigungsmittel kann bis 10 Gew.-%, vorzugsweise 0,1 bis 5, besonders bevorzugt 0,3 bis 3 Gew.-%, bezogen auf die Zusammensetzung des Reinigungsmittels, betragen. Die hydrophilen Emollients können allein oder zu mehreren als Komponente a.) in den erfindungsgemäßen Haut- und Handreinigungsmitteln enthalten sein.

Die als Komponente b.) einsetzbaren Fettalkoholethoxylate weisen vorzugsweise die allgemeine Formel

R-O-(CH2-CH2-O)nH

auf, wobei

R = gesättigter, ungesättigter, verzweigter oder unverzweigter Alkylrest,
n = ganze Zahl von 1 bis 11 sind.

Vorzugsweise werden als gesättigter, ungesättigter, verzweigter oder unverzweigter Alkylrest R =$C_6$ bis $C_{18}$, insbesondere $C_{10}$ bis $C_{16}$ und besonders bevorzugt $C_{11}$ bis $C_{14}$ verwendet, wobei bevorzugt n = 3 bis 6, insbesondere n = 5 bis 7 ist.

In den erfindungsgemäßen Haut- und Handreinigungsmitteln sind 2 bis 40 Gew.-%, vorzugsweise 3 bis 20 und besonders bevorzugt 5 bis 10 Gew.-% Fettalkoholethoxylate, bezogen auf die Zusammensetzung des Reinigungsmittels, enthalten. In einer bevorzugten Ausführungsform enthalten die erfindungsgemäßen Haut- und Handreinigungsmittel 5 bis 10 Gew.-%, bezogen auf die Zusammensetzung des Reinigungsmittels, Laureth-6 als Fettalkoholethoxylat auf.

Bei den auswählbaren Fettalkoholethersulfaten der Komponente b) handelt es sich insbesondere um solche der allgemeinen Formel

$R\text{-}O\text{-}(CH2\text{-}CH2\text{-}O)_nSO_3X$

mit R = einem $C_8$-$C_{18}$, bevorzugt $C_{11}$- $C_{14}$ gesättigten oder ungesättigten, verzweigten oder unverzweigten Alkylrest,
n = einer ganzen Zahl von1 bis 6, vorzugsweise 1 bis 4,
und X = $Na^+$, $NH_4^+$ oder $Mg^{2+}$, wobei Natriumlaurylethersulfat (mit R = $C_{12}$, n = 2 - 3 und X = $Na^+$) besonders bevorzugt wird.

Als Salze von sulfierten und/oder sulfonierten Fettsäuren der Komponente b.) werden erfindungsgemäß Alkalioder Erdalkalisalze von $C_8$-$C_{30}$, bevorzugt $Ci_\theta$-$C_{22}$ Fettsäuren, besonders bevorzugt Rizinusölsulfate, insbesondere $Na^+$ oder $NH_4^+$ Sulfate verwendet. Solche Rizinusölsulfonate sind beispielsweise unter den Marken Monobrilliantöl® (Stockhausen GmbH, Krefeld) oder Standapol SCO® (Henkel KGaA, Düsseldorf) erhältlich.

Die erfindungsgemäßen Haut- und Handreinigungsmittel, die frei von organischen Lösungsmitteln sind, enthalten 30 bis 90, bezogen auf die Zusammensetzung des Reinigungsmittels, bevorzugt 40 bis 80 und besonders bevorzugt 45 bis 75 Gew.-% Wasser als Komponente c).

Obgleich die erfindungsgemäßen Haut- und Handreinigungsmittel eine sehr gute Reinigungswirkung zeigen, so dass der Zusatz von Reinigungsverstärkern entbehrlich ist, können die Haut- und Handreinigungsmittel gegebenenfalls für bestimmte Reinigungsanwendungen Reinigungsverstärker aus der Klasse der Polyole, Polyether, Polyphosphate und Phosphate als Komponente d.) enthalten. Besonders bevorzugt sind hierbei Polyether, die aus der Polymerisation von Ethylenglycol entstehen und 3 bis 20 Einheiten, bevorzugt 4 bis 10 Einheiten. Bevorzugt wird PEG-8 wie es beispielsweise von der Firma Ineos unter den Bezeichnungen PEG 400 bezogen werden können. Desweiteren bevorzugt

sind Polyphosphate, wie das Pentanatriumtripolyphosphat $Na_5P_3O_{10}$ (Natriumtripolyphoshat) sowie die entsprechenden Kalium- und Natriumkalium-Salze, die beispielsweise von der Fa. BK Giulini erhältlich sind.

Die erfindungsgemäßen Haut- und Handreinigungsmittel zeigen eine sehr gute Reinigungswirkung, so dass der Zusatz von Abrasiva zu diesen Reinigungsmitteln entbehrlieh ist. Nichtsdestoweniger können die Haut- und Handreinigungsmittel gegebenenfalls für bestimmte Reinigungsanwendungen Abrasiva als optionale Komponente e.) enthalten. Der Anteil des Abrasivums oder der Abrasiva kann dann 5 bis 30 Gew.-%, bezogen auf die Zusammensetzung des Reinigungsmittels, bevorzugt 5 bis 25 Gew.-%, besonders bevorzugt 5 bis 25 Gew.-% enthalten. Bevorzugt zu verwendende Abrasiva sind beispielsweise Kunststoffreibemittel auf der Basis von Polyethylen oder Polyurethan, Reibemittel auf der Basis von natürlichen Kern- und/oder Schalenmehlen, insbesondere Walnußschalen-, Mandelschalen-, Haselnußschalen-, Olivenkern-, Aprikosenkern- und Kirschkernmehl oder beliebige Gemische dieser Schalen- und Kernmehle und Perlen aus Wachsen, wie z.B. Jojobawachse, wobei mit Wasserstoffperoxid gebleichtes Walnußschalenmehl besonders bevorzugt ist.

Besonders bevorzugt ist der synergistische Einsatz von geringen Mengen an propoxylierten Fettalkoholen als Komponente f.) bis 1,0 Gew.-%, bezogen auf die Zusammensetzung des Reinigungsmittels, mit Komponente a.). Verwendet werden propoxylierte Fettalkohole, die als Anlagerungsprodukte von Propylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technische Mischungen, in Form ihrer Salze, insbesondere Natrium- und/oder Magnesiumsalze erhältlich sind. Bevorzugt ist der Einsatz von Addukten von durchschnittlich 1 bis 20 Mol Propylenoxid an die gewünschten Fettalkohole. Erfindungsgemäß besonders bevorzugt sind propoxylierte Fettalkohole, wie sie beispielsweise von der Fa. Degussa GmbH unter der Bezeichnung VARONIC® APS (PPG-1 1 Stearyl Ether) bzw. VARONIC® APM (PPG-3 Myristyl Ether), erhältlich sind. Eingesetzt werden 0,01 bis 1 Gew.-%, bevorzugt 0,1 bis 0,7 Gew.-%.

Darüber hinaus können die erfindungsgemäßen Haut- und Handreinigungsmittel in einer weiteren Ausführungsform der Erfindung gegebenenfalls eines oder mehrere viskositätsbildende Mittel wie beispielsweise organophile und/oder hydrophile Schichtsilikate als Komponente g.) enthalten, insbesondere Bentonite, Polysaccharide, wie z.B. Cellulose, Guarmehl und/oder Xanthane, modifizierte Polysaccharide, bevorzugt Celluloseether, Carboxyalkylcellulose und/oder Hydroxyalkylcellulosen, vorzugsweise Hydroxyethylcellulose und/oder anorganische Elektrolyte, vorzugsweise Natriumchlorid und/oder Magnesiumsulfat. Erfindungsgemäß besonders bevorzugt sind als Komponente g.) Carboxymethylcellulosen (z.B. Walocel CRT - Wolff Cellulosics, Walsrode), die darüberhinaus die sehr gute schaumstabilisierende Wirkung bei Anwendung der erfindungsgemäßen Haut- und Handreinigungsmittel bewirken. Besonders vorteilhaft enthalten die erfindungsgemäßen Haut- und Handreinigungsmittel 0,1 bis 1,5 Gew.-%, bezogen auf die Gesamtzusammensetzung des Reinigungsmittels.

Weiterhin können die erfindungsgemäßen Haut- und Handreinigungsmittel gegebenenfalls weitere kosmetische Hilfs-, Zusatz- und/oder Wirkstoffe, beispielsweise pH-Regulatoren, Stabilisatoren, vorzugsweise Cetearylalkohol und/oder hydrierte Ricinusöle, wie z.B. Trihydroxystearin, Duftstoffe, Konservierungsmittel, bevorzugt organische Säuren und Antioxidantien, wie z.B. Vitamin E-Acetat als Komponente h.) enthalten. Vorzugsweise können auch ölige oder wässrige Pflegekomponenten wie z.B. Bisabolol, Aloe vera, Panthenol, Sodium PCA, Jojobaöl, Creatin etc. eingesetzt werden, um die Pflegewirkung zu unterstreichen.

Besonders vorteilhaft ist darüberhinaus die Verwendung von alkoxylierten Amiden als Komponente h.), wie z.B. PEG-4 Rapeseedamide (Amidet N - Biesterfeld Spezialche-mie GmbH, Hamburg), vorzugsweise 0,5 bis 5 Gew.-%, besonders bevorzugt 1 bis 4, bezogen auf die Gesamtzusammensetzung des Reinigungsmittels als Stabilisator.

Die Herstellung der erfindungsgemäßen Haut- und Handreinigungsmittel, insbesondere Grobhandreiniger erfolgt üblicherweise mittels bekannter Vorrichtungen im Batch- oder kontinuierlichen Verfahren, wobei die Haut- und Handreinigungsmittel vorzugsweise als cremige Mittel oder als fließfähige viskose Pasten erhalten werden. Geeignete Vorrichtungen sind temperierbare Kessel mit Rührwerk, kontinuierliche Mischer wie Extruder und Dispergatoren.

Es wurde überraschender Weise gefunden, dass es trotz des Verzichtes auf den Einsatz von hydrophoben Emollients wie z.B. pflanzlicher Öle im Falle der Grobhandreiniger gemäß DE 197 48 921 C2 durch die Verwendung hydrophiler Emollients entsprechend Komponente a.) in den erfindungsgemäßen Haut- und Handreinigungsmitteln eine deutlich gesteigerte Hautpflegewirkung sowie eine verbesserte Hautanmutung beobachtet werden kann, ohne dass es hierdurch zu einer Beeinträchtigung der Reinigungswirkung kommt.

Die erfindungsgemäßen Haut- und Handreinigungsmittel zeigen eine sehr gute Reinigungswirkung, bei gleichzeitig sehr guter Hautverträglichkeit und geringer Hautaustrocknung. Besonders vorteilhaft ist, dass die erfindungsgemäßen Haut- und Handreinigungsmittel während des Waschens ein sehr gutes Hautgefühl erzeugt. Darüberhinaus konnte beobachtet werden, dass selbst unter leichter Ölbelastung die erfindungsgemäßen Haut- und Handreinigungsmittel deutlich stärker schäumen als Grobhandreiniger des Standes der Technik.

Das Hautgefühl nach dem Waschen ist auch nach einiger Zeit deutlich angenehmer aufgrund der geringeren Hautaustrocknung.

Die Erfindung wird durch folgende Beispiele und Untersuchungen, wie Hautverträglichkeitsprüfung mit Hilfe des Duhring-Kammer-Testes, Hautaustrocknung mit Hilfe des Corneometers und Reinigungskraft mit Hilfe des Handwaschtests beschrieben.

Prüfmethoden

Bestimmung des HLB-Wertes

Der HLB-Wert wird bestimmt, in dem eine 5 %-ige Dispersion/Lösung der zu prüfenden Substanz, hier des hydrophilen Emollients, in Wasser angesetzt und die Wasserlöslichkeit bestimmt wird. Die Beurteilung erfolgt optisch: Trübung, Löslichkeit, Dispersionsgrad. Der HLB-Wert wird ermittelt durch den Vergleich mit Lösungen von Emulgatoren, deren HLB-Wert bekannt ist.

Ermittelte HLB-Werte:

- TEGOSOFT LSE 65 K soft HLB: 12
- TEGOSOFT PC 31 HLB: 14
- TEGOSOFT PC 41 HLB: 16
- Lamesoft PO 65 HLB: 9

HLB-Werte gemäß Hersteller:

- Glycerox HE HLB: 11

Duhring-Kammer Test

Die Hautverträglichkeitsprüfung erfolgt mit Hilfe des Duhring-Kammer-Testes nach PJ. Frosch, A.M. Kligman, Contact Dermatities 5, Seite 73, 1979; modifiziert nach J. Kresken, W.S. Wassilew, H+G4, Seite 334, 1992; A. Klotz, Am. J. Cont. Derm., 2001, 12 (1), Seite 52 und A. Klotz, M. Veeger, Pharm. Ztg. 2000, 145 (35), Seite 47 bis 51.

Bei dieser Methode handelt es sich um ein in vivo-Testmodell zur Überprüfung der Hautverträglichkeit verschiedener Testprodukte im direkten Vergleich. Den Probanden werden die zu testenden Produkte in luftundurchlässigen Aluminiumkammern auf die volare Seite der Unterarme an 4 Tagen hintereinander auf jeweils das gleiche Testareal appliziert. Beurteilt werden die entstandenen Hautirritationen nach unten angegebener Skala bzw. die Applikationszeit 1 Tag nach der letzten Produktapplikation.

R = Rötung (Erythem): 0 = kein Erythem 4 = starkes Erythem
S = Schuppung: 0 = keine Schuppung 4 = starke Schuppung
F = Fissuren: 0 = keine Fissuren 3 = starke Fissuren

Als Beurteilungskriterien ergeben sich die

1. Irritation x als Mittelwert aus der Summe der Irritationswerte von R, S und F der n Probanden
2. Applikationszeit h als Mittelwert der tolerierten Applikationszeit in Stunden der n Probanden.

Aus diesen beiden Werten läßt sich die relative Hautverträglichkeit (A-Wert) nach folgender Formel berechnen:

$$\overline{A} = \frac{\overline{h}}{\sqrt{\overline{x}}}$$

Somit ist es möglich, eine Relation zwischen der Applikationszeit und der während dieser Zeit entstandenen Irritation mit einem Wert zu beschreiben. Als Hilfe zur Beurteilung der Hautverträglichkeit der getesteten Produkte läßt sich die folgende Tabelle heranziehen:

A-Wert:

> 23 exzellente Hautverträglichkeit
18 - 23 sehr gute Hautverträglichkeit

13 - 18 gute Hautverträglichkeit
8 - 13 befriedigende Hautverträglichkeit
3 - 8 ausreichende Hautverträglichkeit
< 3 mangelhafte Hautverträglichkeit

**Prüfung der Hautaustrocknung mit Hilfe des Corneometers**

Die Corneometermessung ist ein non-invasives kapazitives Meßverfahren zur Beurteilung des Feuchtigkeitszustandes der obersten Hautschichten des Stratum corneums. Dieser wird beim hautgesunden Probanden zum größten Teil durch externe Einflüsse bestimmt.

Häufige Hautreinigung führt zu einer Hautentfettung und dadurch zu einer Feuchtigkeitsabnahme in den obersten Hautschichten. Die Abnahme des Feuchtigkeitsgehaltes der Haut stellt eine Verschlechterung des Hautzustandes dar und bedingt ein erhöhtes Ekzemrisiko. Dieser Effekt kann mit dem Corneometer z.B. dem CM 825 der Firma Courage & Khazaka Electronic GmbH, Köln, meßtechnisch erfaßt werden.

Vor Testbeginn werden die Ausgangswerte (Co) bestimmt und bei Testende die Endwerte ermittelt ($C_n$).
Die Änderung der Hautfeuchte C berechnet sich wie folgt:

$$C = (C_n - C_0)$$

Negative Werte bedeuten eine Erhöhung der Hautfeuchte, positive Werte bedeuten eine Verringerung der Hautfeuchtigkeit.

**Unterarmwaschtest (UWT)**

Der Unterarmwaschtest (UWT) dient zur Prüfung der Hautverträglichkeit von Syndetpräparaten und Handreinigern. Besonders gut geeignet ist dieser Test für die Beurteilung von reibemittelhaltigen Testprodukten. Aufgrund der mechanisch verursachten Reibung am Unterarm lassen sich die verschiedenen Reibemittel und Reibemittelkonzentrationen auf ihre Hautreizung prüfen.

Der Test ist praxisnah aufgebaut und kann an einen Duhring-Kammer-Test zur Verifizierung der Ergebnisse angeschlossen werden.
An vier aufeinanderfolgenden Tagen werden kontrollierte Waschungen mit den Prüfprodukten (0,5g - 1 g bzw. 0,5 ml - 1 ml Syndetpräparate oder reibemittelhaltige Handreiniger) an der empfindlichen Haut des volaren Unterarms durchgeführt.
Das Testareal wird mit Leitungswasser befeuchtet und anschließend das zu prüfende Produkt auf die Haut gebracht und insgesamt 2 Minuten lang auf dem Unterarm großflächig einmassiert. Die anderen Felder werden in der gleichen Weise mit dem jeweiligen Prüfprodukt behandelt. Die Beurteilung der Hautreaktionen wird täglich vor der 1.waschung und am Tag 5 vorgenommen.
Die Bewertung erfolgt visuell und wird ggf. unterstützt durch objektive nicht invasive Messverfahren (z.B. TEWL, Corneometer, D-Squames®).

0 = keine Reaktion
1 = schwache fleckige(punktförmige) Rötung, leichte Schuppung
2 = mäßige bis mittelstarke Rötung, leicht rauhe Haut, mittlere Schuppung 3 = starke mittelflächige Rötung, sehr rauhe Haut, deutliche Schuppung,

punktierte Erosionen

**Prüfung der Reinigungskraft mit Hilfe des Handwaschtests**

Das Testmodell des Handwaschtests mit standardisiertem Schmutz oder Lack gibt Auskunft über die Reinigungswirkung der zu prüfenden Produkte. Für eine Praxisrelevanz ist es erforderlich, dass alle Probanden eine charakteristische, durch manuelle Arbeit bedingte Hautstruktur der Handinnenflächen besitzen. Morgens und nachmittags wird mit je einem Produkt folgender Test durchgeführt:

**Testdurchführung mit Wasser:**

- 0,5 g Schmutz (Modellverschmutzung, Praxisschmutz oder Lack) werden auf die Handinnenfläche und auf den Handrücken verteilt und verrieben
- 1,5 min. trocknen lassen
- 1,2 g Reinigungsmittel werden aufgetragen und eingerieben
- 1 ml Wasser wird zugefügt und 30 s gewaschen
- nochmals 1 ml Wasser zufügen und 30 s waschen lassen
- abspülen unter fließendem kalten Wasser
- visuelle Beurteilung der Restverschmutzung (RV) auf dem Handrücken und der Handinnenfläche gemäß Skala s.u.

**Testdurchführung ohne Wasser:**

- 0,5 g Schmutz (Modellverschmutzung, Praxisschmutz oder Lack) werden auf die Handinnenfläche und auf den Handrücken verteilt und verrieben
- 1,5 min. trocknen lassen
- 1,2 g Reinigungsmittel werden aufgetragen und verrieben
- mit einem Cellulose Papier werden die Verschmutzung auf den Handflächen mit dem Produkt zusammen entfernt.
- visuelle Beurteilung der Restverschmutzung (RV) auf dem Handrücken und der Handinnenfläche gemäß Skala s.u.

0 = sauber 5 = kein Reinigungseffekt (Abstufung in 0,5er Schritten möglich)

Die Berechnung des prozentualen Reinigungseffektes erfolgt nach folgender Formel:

$$\text{Reinigungseffekt [\%]} = \frac{10 - (\overline{RV}_{\text{innen}} + \overline{RV}_{\text{außen}})}{10} * 100\%$$

RV innen = Mittelwert der Restverschmutzung Handinnenflächen von n Meßreihen (Probanden)
RV außen = Mittelwert der Restverschmutzung Handaußenflächen von n Meßreihen (Probanden)

Da die Bestimmung der Reinigungswirkung infolge der Testmethode eine höhere Schwankungsbreite aufweist, ist eine Absolutabweichung von 5 % zwischen zwei Meßreihen zulässig.

Zusammensetzung einer geeigneten Modellverschmutzung:

Motoröl: 54,15 %
Vaseline: 18,05 %
Wollwachs: 18,05 %
Graphit: 3,61 %
Flammruß: 5,42 %
Eisenoxid ($Fe_2O_3$): 0,72 %

**Ausführungsbeispiele:**

Es wurden Haut- und Handreinigungsmittel gemäß den in Tabelle 1 angegebenen Zusammensetzungen durch Zusammenrühren aller Komponenten mittels der in der Kosmetik üblichen Kalt-Kalt-, Heiß-Kalt- oder Heiß-Heiß-Verfahren bis maximal 70° C hergestellt. Die Mittel wurden im Hinblick auf ihre Hautverträglichkeit, Hautaustrocknung und Reinigungswirkung gegenüber einem Modellschmutz und Lack charakterisiert.

Die erfindungsgemäßen Beispiel-Rezepturen A bis P weisen in dem beschriebenen Hautverträglichkeitstest im Vergleich zur lösemittelfreien Vergleichsrezeptur T, die entsprechend der DE 197 48 921 C2 hergestellt wurde, eine vergleichbare, sehr gute Hautverträglichkeit und im Corneometertest eine verringerte Hautaustrocknung nach der Reinigung auf. Vergleichsrezepturen S, die entsprechend der 199 16 036 A1 hergestellt wurden, zeigten deutlich schlechtere Hautverträglichkeit und höhere Hautaustrocknungswerte gemessen nach UWT als die Rezepturen A bis J.

Darüberhinaus zeigten die erfindungsgemäßen Rezepturen A bis J in dem beschriebenen, standardisierten Reinigungstest im Vergleich N gegenüber einer Lackverschmutzung und gegenüber einer Modellverschmutzung eine

vergleichbare sehr gute Reinigungswirkung.

## Patentansprüche

1. Haut- und Handreinigungsmittel, insbesondere Grobhandreiniger, **dadurch gekennzeichnet, dass** sie einen Gehalt von mindestens 0,1 Gew.-% von wenigstens einem hydrophilen Emollient mit einem HLB-Wert von ≥10 aufweisen und dass sie einen Gehalt, jeweils bezogen auf die Gesamtzusammensetzung des Reinigungsmittels, von den Komponenten

    a.) mindestens 0,1 Gew.-% von wenigstens einem Polyolester als hydrophilem Emollient mit einem HLB-Wert von ≥10,
    b.) 2 bis 40 Gew.-% wenigstens ein Tensid ausgewählt aus der Gruppe der Fettalkoholethoxylate, Fettalkoholethersulfate und Salze sulfierter und/oder sulfonierter Fettsäuren, **gekennzeichnet dadurch dass** das Mittel wenigstens ein Fettalkoholethoxylat enthält,
    c.) 30 bis 90 Gew.-% Wasser,
    d.) 0 bis 30 Gew.-% eines oder mehrerer Reinigungsverstärker ausgewählt aus der Gruppe der Polyole, Polyether, Polyphosphate und Phosphate,
    e.) 5 bis 30 Gew.-% eines oder mehrerer Abrasiva,
    f.) 0 bis 1,0 Gew.-% propoxylierte Fettalkohole,
    g.) gegebenenfalls ein oder mehrere viskositätsbildende Mittel,
    h.) gegebenenfalls weitere kosmetische Hilfs-, Zusatz- und/oder Wi rkstoffe,

    wobei die Summe der Komponenten a.) bis h.) 100 Gew. % ergibt, aufweisen.

2. Haut- und Handreinigungsmittel nach Anspruch 1, **dadurch gekennzeichnet, dass** sie einen Gehalt an Komponente a.) von 0,1 bis 10 Gew.-% aufweisen.

3. Haut- und Handreinigungssystem nach Anspruch 1 oder 2, **dadurch gekennzeichnet, dass** Komponente a.) wenigstens ein Polyglycerylpartialester der allgemeinen Formel

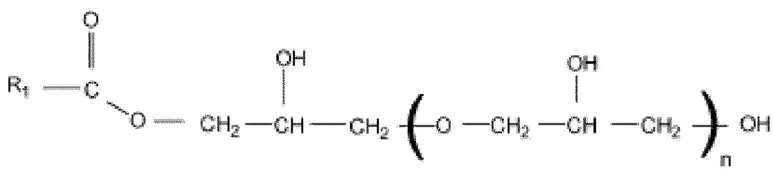

    ist, wobei

    $R_1$ = linearer, verzweigter oder cyclischer, gesättigter oder ungesättigter Alkyl- bzw. Alkenylrest mit 6 bis 18 Kohlenstoffatomen und
    n = ganze Zahl von 1 bis 9 sind,
    und/oder Polyglycerinester aufweisen.

4. Haut- und Handreinigungsmittel nach einem der Ansprüche 1 bis 3, **dadurch gekennzeichnet, dass** Komponente a.) ein Polyglycerylpartialester ist.

5. Haut- und Handreinigungsmittel nach einem der Ansprüche 1 bis 4, **dadurch gekennzeichnet, dass** es 0,01 bis 1,0 Gew.-% eines propoxylierten Fettalkohols als Komponente f.) enthält.

6. Haut- und Handreinigungsmittel nach einem der Ansprüche 1 bis 5, **dadurch gekennzeichnet, dass** als Komponente a.) Polyglyceryl-3-caprat und als Komponente f.) PPG-11 Stearyl Ether verwendet wird.

7. Haut- und Handreinigungsmittel nach einem der Ansprüche 1 bis 6, **dadurch gekennzeichnet, dass** Komponente b.) wenigstens ein Fettalkoholethoxylat der allgemeinen Formel

    R-O-(CH2-CH2-0)nH

ist, wobei

R = gesättigter, ungesättigter, verzweigter oder unverzweigter Alkylrest,
n = ganze Zahl von 1 bis 11 sind.

8. Haut- und Handreinigungsmittel nach einem der Ansprüche 1 bis 7, **dadurch gekennzeichnet, dass** es 5 bis 25 Gew.% eines oder mehrerer Abrasiva als Komponente e.) enthält.

9. Haut- und Handreinigungsmittel nach Anspruch 8, **dadurch gekennzeichnet, dass** 5 bis 20 Gew.-% eines oder mehrerer Abrasiva als Komponente e.) enthält.

10. Haut- und Handreinigungsmittel nach einem der Ansprüche 1 bis 9, **dadurch gekennzeichnet, dass** Komponente b.) weiterhin wenigstens ein Tensid ausgewählt aus der Gruppe der Fettalkoholethersulfate und Salze sulfierter und/oder sulfonierter Fettsäuren enhält.

11. Haut- und Handreinigungsmittel nach einem der Ansprüche 1 bis 10, **dadurch gekennzeichnet, dass** es als Abrasiva der Komponente e.) Kunststoffreibemittel auf der Basis von Polyethylen oder Polyurethan, Reibemittel auf der Basis von natürlichen Kern- und/oder Schalenmehlen, oder Gemische dieser Schalen- und Kernmehle und /oder Perlen aus Wachsen enhält.

**Claims**

1. Skin and hand cleanser, in particular a course cleanser for the hands, **characterised in that** it contains at least 0.1 wt.% of at least one hydrophilic emollient which has an HLB value of ≥10, and **in that** it contains, in each case based on the total composition of the cleanser, the components

a.) at least 0.1 wt.% of at least one polyolester as a hydrophilic emollient which has an HLB value of ≥10,
b.) 2 to 40 wt.% of at least one surfactant selected from the group of fatty alcohol ethoxylates, fatty alcohol ether sulphates and salts of sulphated and/or sulphonated fatty acids, **characterised in that** the cleanser contains at least one fatty alcohol ethoxylate,
c.) 30 to 90 wt.% of water,
d.) 0 to 30 wt.% of one or more cleaning boosters selected from the group of polyols, polyethers, polyphosphates and phosphates,
e.) 5 to 30 wt.% of one or more abrasives,
f.) 0 to 1.0 wt.% of propoxylated fatty alcohols,
g.) optionally one or more viscosity-enhancing agents,
h.) optionally further cosmetic auxiliary substances, additives and/or active ingredients,

the sum of components a.) to h.) equating to 100 wt.%.

2. Skin and hand cleanser according to claim 1, **characterised in that** it contains component a.) in an amount of from 0.1 to 10 wt.%.

3. Skin and hand cleansing system according to either claim 1 or claim 2, **characterised in that** component a.) is at least one polyglyceryl partial ester of the general formula

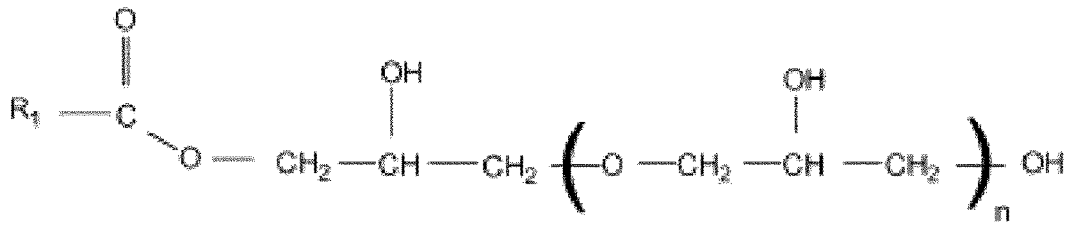

in which

$R_1$ = a linear, branched or cyclical, saturated or unsaturated alkyl or alkenyl functional group comprising 6 to 18 carbon atoms, and
n = an integer from 1 to 9,
and/or have polyglycerol esters.

**4.** Skin and hand cleanser according to any one of claims 1 to 3, **characterised in that** component a.) is a polyglyceryl partial ester.

**5.** Skin and hand cleanser according to any one of claims 1 to 4, **characterised in that** it contains 0.01 to 1.0 wt.% of a propoxylated fatty alcohol as component f.).

**6.** Skin and hand cleanser according to any one of claims 1 to 5, **characterised in that** polyglyceryl-3-caprate is used as component a.) and PPG-11 stearyl ether is used as component f.).

**7.** Skin and hand cleanser according to any one of claims 1 to 6, **characterised in that** component b.) is at least one fatty alcohol ethoxylate of the general formula

R-O-(CH2-CH2-0)nH,

in which

R = a saturated, unsaturated, branched or unbranched alkylfunctional group, and
n = an integer from 1 to 11.

**8.** Skin and hand cleanser according to any one of claims 1 to 7, **characterised in that** it contains 5 to 25 wt.% of one or more abrasives as component e.).

**9.** Skin and hand cleanser according to claim 8, **characterised in that** it contains 5 to 20 wt.% of one or more abrasives as component e.).

**10.** Skin and hand cleanser according to any one of claims 1 to 9, **characterised in that** component b.) further contains at least one surfactant selected from the group of fatty alcohol ether sulphates and salts of sulphated and/or sulphonated fatty acids.

**11.** Skin and hand cleanser according to any one of claims 1 to 10, **characterised in that** it contains plastics abrasive agents based on polyethylene or polyurethane, abrasive agents based on natural kernel flours and/or shell flours, or mixtures of these shell flours and kernel flours and/or wax beads as the abrasive of component e.).

## Revendications

**1.** Produits nettoyants pour la peau et les mains, en particulier produits destinés au nettoyage grossier des mains, **caractérisés en ce qu'**ils présentent une teneur d'au moins 0,1 % en poids d'au moins un émollient hydrophile d'une valeur de HLB ≥ 10, et qu'ils présentent une teneur, respectivement rapportée à la composition totale du produit nettoyant, des composants suivants

a.) au moins 0,1 % en poids d'au moins un ester de polyol en tant qu'émollient hydrophile présentant une valeur de HLB ≥ 10,
b.) 2 à 40 % en poids d'au moins un tensioactif choisi parmi le groupe des éthoxylates d'alcool gras, des éthersulfates d'alcool gras et des sels d'acides gras sulfurisés et/ou sulfonés, **caractérisés en ce que** le produit contient au moins un éthoxylate d'alcool gras,
c.) 30 à 90 % en poids d'eau,
d.) 0 à 30 % en poids d'un ou de plusieurs agents de renforcement de nettoyage choisis parmi le groupe des polyols, des polyéthers, des polyphosphates et des phosphates,
e.) 5 à 30 % en poids d'un ou de plusieurs abrasifs,
f.) 0 à 1,0 % en poids d'alcools gras propoxylés,
g.) éventuellement un ou plusieurs produits conférant de la viscosité,
h.) éventuellement d'autres excipients, adjuvants et/ou principes actifs cosmétiques, dans lesquels la somme

des composants a.) à h.) est égale à 100 % en poids,

**2.** Produits nettoyants pour la peau et les mains selon la revendication 1, **caractérisés en ce qu'**ils présentent une teneur en composant a.) allant de 0,1 à 10 % en poids.

**3.** Produits nettoyants pour la peau et les mains selon la revendication 1 ou 2, **caractérisés en ce que** le composant a.) est au moins un ester partiel de polyglycéryle de la formule générale

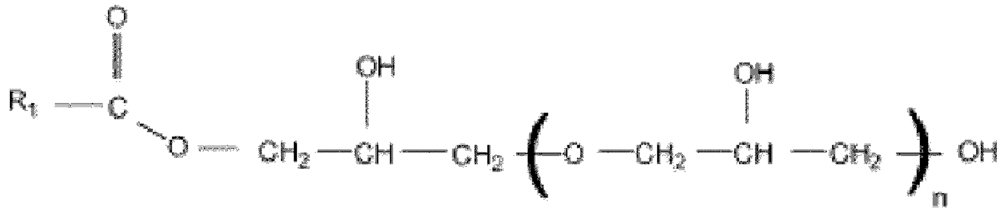

dans lesquels

$R_1$ est un radical alkyle ou alcényle linéaire, ramifié ou cyclique, saturé ou insaturé comprenant 6 à 18 atomes de carbone, et
n est un nombre entier allant de 1 à 9,
et/ou présentent un ester de polyglycérol.

**4.** Produits nettoyants pour la peau et les mains selon l'une quelconque des revendications 1 à 3, **caractérisés en ce que** le composant a.) est un ester partiel de polyglycéryle.

**5.** Produit nettoyant pour la peau et les mains selon l'une quelconque des revendications 1 à 4, **caractérisé en ce qu'**il contient en tant que composant f.) 0,01 à 1,0 % en poids d'un alcool gras propoxylé.

**6.** Produits nettoyants pour la peau et les mains selon l'une quelconque des revendications 1 à 5, **caractérisés en ce qu'**est utilisé en tant que composant a.) du polyglycéryl-3-caprate et en tant que composant f.) du PPG-11 éther stéarylique.

**7.** Produits nettoyants pour la peau et les mains selon l'une quelconque des revendications 1 à 6, **caractérisés en ce que** le composant b.) est au moins un éthoxylate d'alcool gras de la formule générale

R-O-(CH2-CH2-O)nH,

dans lesquels

R est un radical alkyle saturé, insaturé, ramifié ou non ramifié,
n est un nombre entier allant de 1 à 11.

**8.** Produit nettoyant pour la peau et les mains selon l'une quelconque des revendications 1 à 7, **caractérisé en ce qu'**il contient en tant que composant e.) 5 à 25 % en poids d'un ou de plusieurs abrasifs.

**9.** Produits nettoyants pour la peau et les mains selon la revendication 8, **caractérisés en ce qu'**ils contiennent en tant que composant e.) 5 à 20 % en poids d'un ou de plusieurs abrasifs.

**10.** Produits nettoyants pour la peau et les mains selon l'une quelconque des revendications 1 à 9, **caractérisés en ce que** le composant b.) contient par ailleurs au moins un tensioactif choisi parmi le groupe des éthersulfates d'alcool gras et des sels d'acides gras sulfurisés et/ou sulfonés.

**11.** Produit nettoyant pour la peau et les mains selon l'une quelconque des revendications 1 à 10, **caractérisé en ce qu'**il contient en tant qu'abrasif du composant e.) des agents de frottement en plastique à base de polyéthylène ou de polyuréthane, des agents de frottement à base de farines de noyaux et/ou d'écales naturelles ou des mélanges desdites farines d'écales et/ou de noyaux et/ou des perles de cires.

Fig. 1

| **Inhaltsstoffe nach INCI-Nomenklatur** | **A** | **B** | **C** | **D** | **E** | **F** | **G** | **H** | **I** |
|---|---|---|---|---|---|---|---|---|---|
| AQUA(WATER) | 57,3 | 60,5 | 60,2 | 57,0 | 58,9 | 48,7 | 65,8 | 71,4 | 76,0 |
| JUGLANS REGIA SHELL POWDER | 15,0 | 15,0 | 15,0 | 15,0 | 15,0 | 15,0 | 12,0 | 6,0 | 0,0 |
| QUARZSAND | | | | | | | | | |
| Natriumcarbonate | | | | | | | | | |
| Rape seed oil | | | | | | | | | |
| | | | | | | | | | |
| Natriumlaurylsulfat | | | | | | | | | |
| LAURETH-6 | 9,0 | 6,0 | 6,0 | 9,9 | | | 6,0 | 6,0 | 6,0 |
| LAURETH-5 | | | | | | 9,0 | | | |
| POLAXAMER 407 (EO/PO Blockpolymer- Pluronic) | | | | | 6,0 | | | | |
| ALKYLPOYGLUCOSID | | | | | | | | | |
| SODIUM LAURETH SULFATE | 3,1 | 3,1 | 3,1 | 3,1 | 5,0 | 3,0 | 3,3 | 3,3 | 6,0 |
| SULFATED CASTOR OIL | 2,8 | 2,8 | 2,8 | 2,8 | 2,5 | 2,5 | 2,8 | 2,8 | 4,0 |
| DISODIUM LAURETH SULFOSUCCINATE | 3,0 | 2,1 | 2,1 | 2,4 | 1,0 | 3,0 | 1,8 | 1,8 | 1,5 |
| SODIUM COCOAMPHOACETAT | | | | | 2,0 | | | | |
| DISODIUM PEG 4 COCAMIDO MIPA-SULFOSUCCINAT | | | | | | 1,0 | | | |
| Cocoamidoproylbetain | | | | | | | | | |
| COCAMIDE DEA | | | | | | | | | |
| PEG-4 RAPESEEDAMIDE | 2,0 | 1,5 | 1,5 | 1,5 | 1,5 | 2,0 | 2,0 | 2,0 | 1,5 |
| | | | | | | | | | |
| SODIUM TRIPOLYPHOSHATE | 1,7 | 2,5 | 2,5 | 2,5 | 2,5 | | | | |
| PEG-8 | | | | | | 10,0 | | | |
| | | | | | | | | | |
| POLYGLYCERYL-3 CAPRATE (Tegosoft PC 31) | **1,5** | | **1,5** | **1,5** | **1,0** | **1,2** | **1,2** | **1,2** | **1,5** |
| POLYGLYCERYL-4 CAPRATE (Tegosoft PC 41) | | | | | | | | | |
| SUCROSE COCOATE Tegosoft LSE-65Ksoft) | | **1,5** | | | | | | | |
| PPG-11 STEARYL ETHER (Varonic APS) | **0,3** | | **0,3** | **0,3** | | **0,3** | **0,3** | **0,3** | **0,2** |
| PPG-3 MYRISTYL ETHER (Varonic APM) | | | | | **0,3** | | | | |
| Coco-Glucoside and Glyceryl Oleate (Lamesoft PO 65) | | | | | | | | | |
| | | | | | | | | | |
| CARBOXYMETHYL CELLULOSE | 0,7 | 0,6 | 0,6 | 0,6 | 0,7 | 0,7 | 0,7 | 0,7 | 0,7 |
| XANTHAN GUM | | | | | 0,3 | | | | |
| Stearinsäure | | | | | | | | | |
| SODIUM CHLORIDE | 0,6 | 1,5 | 1,5 | 0,5 | 0,5 | 0,6 | 1,5 | 1,5 | 0,6 |
| Oleic Acid | | | | | | | 0,6 | 0,9 | |
| | | | | | | | | | |
| SODIUM BENZOATE | 0,6 | 0,6 | 0,6 | 0,6 | 0,6 | 0,6 | 0,6 | 0,6 | 0,6 |
| POTASSIUM SORBATE | 0,3 | 0,3 | 0,3 | 0,3 | 0,3 | 0,3 | 0,3 | 0,3 | 0,3 |
| PARFUM (FRAGRANCE) | 0,2 | 0,2 | 0,2 | 0,2 | 0,2 | 0,2 | 0,2 | 0,2 | 0,2 |
| CITRIC ACID | 1,4 | 1,3 | 1,3 | 1,3 | 1,5 | 1,4 | 0,5 | 0,5 | 0,5 |
| NAOH | | | | | | | | | |
| CI 77891 (Titandioxid) | 0,5 | 0,5 | 0,5 | 0,5 | 0,2 | 0,5 | 0,5 | 0,5 | 0,4 |

| Inhaltsstoffe nach INCI-Nomenklatur | J | K | L | M | N | O | P |
|---|---|---|---|---|---|---|---|
| AQUA(WATER) | 79,5 | 30,6 | 30,6 | 65,8 | 68,5 | 64,8 | 75,2 |
| JUGLANS REGIA SHELL POWDER | 0,0 | 30,0 | 20,0 | 12,0 | 8,0 | 12,0 | 0,0 |
| QUARZSAND | | | | | | | |
| Natriumcarbonate | | | | | | | |
| Rape seed oil | | | | | | | |
| | | | | | | | |
| Natriumlaurylsulfat | | | | | | | |
| LAURETH-6 | 3,0 | 20,0 | 2,0 | 6,0 | 9,0 | 9,0 | |
| LAURETH-5 | | | | | | | 6,0 |
| POLAXAMER 407 (EO/PO Blockpolymer- Pluronic) | | | | | | | |
| ALKYLPOYGLUCOSID | | | | | | | |
| SODIUM LAURETH SULFATE | 6,0 | 6,0 | 30,0 | 3,3 | 3,3 | 3,3 | 5,0 |
| SULFATED CASTOR OIL | 4,0 | 4,0 | 8,0 | 2,8 | 2,8 | 2,8 | 2,5 |
| DISODIUM LAURETH SULFOSUCCINATE | 1,5 | | | 1,8 | 1,8 | 1,8 | 3,0 |
| SODIUM COCOAMPHOACETAT | | | | | | | |
| DISODIUM PEG 4 COCAMIDO MIPA-SULFOSUCCINAT | | | | | | | |
| Cocoamidoproylbetain | | | | | | | |
| COCAMIDE DEA | | | | | | | |
| PEG-4 RAPESEEDAMIDE | 2,5 | 2,5 | 2,5 | 2,0 | 1,5 | 1,5 | 3,5 |
| | | | | | | | |
| SODIUM TRIPOLYPHOSHATE | | | | | | | |
| PEG-8 | | | | | | | |
| | | | | | | | |
| **POLYGLYCERYL-3 CAPRATE (Tegosoft PC 31)** | **0,2** | **3,0** | **3,0** | | **1,5** | | **1,2** |
| **POLYGLYCERYL-4 CAPRATE (Tegosoft PC 41)** | | | | | | **1,2** | |
| **SUCROSE COCOATE Tegosoft LSE-65Ksoft)** | | | | **1,5** | | | |
| **PPG-11 STEARYL ETHER (Varonic APS)** | | **0,6** | **0,6** | | | | **0,3** |
| **PPG-3 MYRISTYL ETHER (Varonic APM)** | | | | | **0,3** | **0,3** | |
| **Coco-Glucoside and Glyceryl Oleate (Lamesoft PO 65)** | | | | | | | |
| | | | | | | | |
| CARBOXYMETHYL CELLULOSE | 0,7 | 0,7 | 0,7 | 0,7 | 0,7 | 0,7 | 0,7 |
| XANTHAN GUM | | | | | | | |
| Stearinsäure | | | | | | | |
| SODIUM CHLORIDE | 0,6 | 0,6 | 0,6 | 1,5 | 0,6 | 0,6 | 0,6 |
| Oleic Acid | | | | 0,6 | | | |
| | | | | | | | |
| SODIUM BENZOATE | 0,6 | 0,6 | 0,6 | 0,6 | 0,6 | 0,6 | 0,6 |
| POTASSIUM SORBATE | 0,3 | 0,3 | 0,3 | 0,3 | 0,3 | 0,3 | 0,3 |
| PARFUM (FRAGRANCE) | 0,2 | 0,2 | 0,2 | 0,2 | 0,2 | 0,2 | 0,2 |
| CITRIC ACID | 0,5 | 0,5 | 0,5 | 0,5 | 0,5 | 0,5 | 0,5 |
| NAOH | | | | | | | |
| CI 77891 (Titandioxid) | 0,4 | 0,4 | 0,4 | 0,4 | 0,4 | 0,4 | 0,4 |

| Inhaltsstoffe nach INCI-Nomenklatur | Q zu B | R zu G | S | T | U |
|---|---|---|---|---|---|
| AQUA(WATER) | 59,0 | 64,9 | 35,7 | 40,9 | 56,6 |
| JUGLANS REGIA SHELL POWDER | 15,0 | 12,0 | | 13,0 | 15,0 |
| QUARZSAND | | | 35,0 | | |
| Natriumcarbonate | | | 0,5 | | |
| Rape seed oil | | | | 20 | 10 |
| | | | | | |
| Natriumlaurylsulfat | | | 20,0 | | |
| LAURETH-6 | 6,0 | 6,0 | | 11,0 | 7,0 |
| LAURETH-5 | | | | | |
| POLAXAMER 407 (EO/PO Blockpolymer- Pluronic) | | | | | |
| ALKYLPOYGLUCOSID | | | 0,6 | | |
| SODIUM LAURETH SULFATE | 3,1 | 3,3 | 0,8 | 7,5 | 3,0 |
| SULFATED CASTOR OIL | 2,8 | 2,8 | | 2,1 | 3,5 |
| DISODIUM LAURETH SULFOSUCCINATE | 2,1 | 1,8 | | | |
| SODIUM COCOAMPHOACETAT | | | | | |
| DISODIUM PEG 4 COCAMIDO MIPA-SULFOSUCCINAT | | | | | |
| Cocoamidoproylbetain | | | 0,5 | | |
| COCAMIDE DEA | | | 0,5 | | |
| PEG-4 RAPESEEDAMIDE | 1,5 | 2,0 | | | |
| | | | | | |
| SODIUM TRIPOLYPHOSHATE | 2,5 | | | | |
| PEG-8 | | | | | |
| | | | | | |
| **POLYGLYCERYL-3 CAPRATE (Tegosoft PC 31)** | | | | | |
| **POLYGLYCERYL-4 CAPRATE (Tegosoft PC 41)** | | | | | |
| **SUCROSE COCOATE Tegosoft LSE-65Ksoft)** | | | | | |
| **PPG-11 STEARYL ETHER (Varonic APS)** | | | | | |
| **PPG-3 MYRISTYL ETHER (Varonic APM)** | | | | | |
| **Coco-Glucoside and Glyceryl Oleate (Lamesoft PO 65)** | **3,0** | **2,4** | **5,0** | | |
| | | | | | |
| CARBOXYMETHYL CELLULOSE | 0,6 | 0,7 | | 0,7 | 0,7 |
| XANTHAN GUM | | | | 0,3 | 0,3 |
| Stearinsäure | | | 1,1 | | |
| SODIUM CHLORIDE | 1,5 | 1,5 | | 2,4 | 0,6 |
| Oleic Acid | | 0,6 | | | 1,2 |
| | | | | | |
| SODIUM BENZOATE | 0,6 | 0,6 | | 0,6 | 0,6 |
| POTASSIUM SORBATE | 0,3 | 0,3 | | 0,3 | 0,3 |
| PARFUM (FRAGRANCE) | 0,2 | 0,2 | 0,2 | 0,2 | 0,2 |
| CITRIC ACID | 1,3 | 0,5 | | 0,6 | 0,6 |
| NAOH | | | 0,2 | | |
| CI 77891 (Titandioxid) | 0,5 | 0,5 | | 0,4 | 0,4 |

Fig. 2

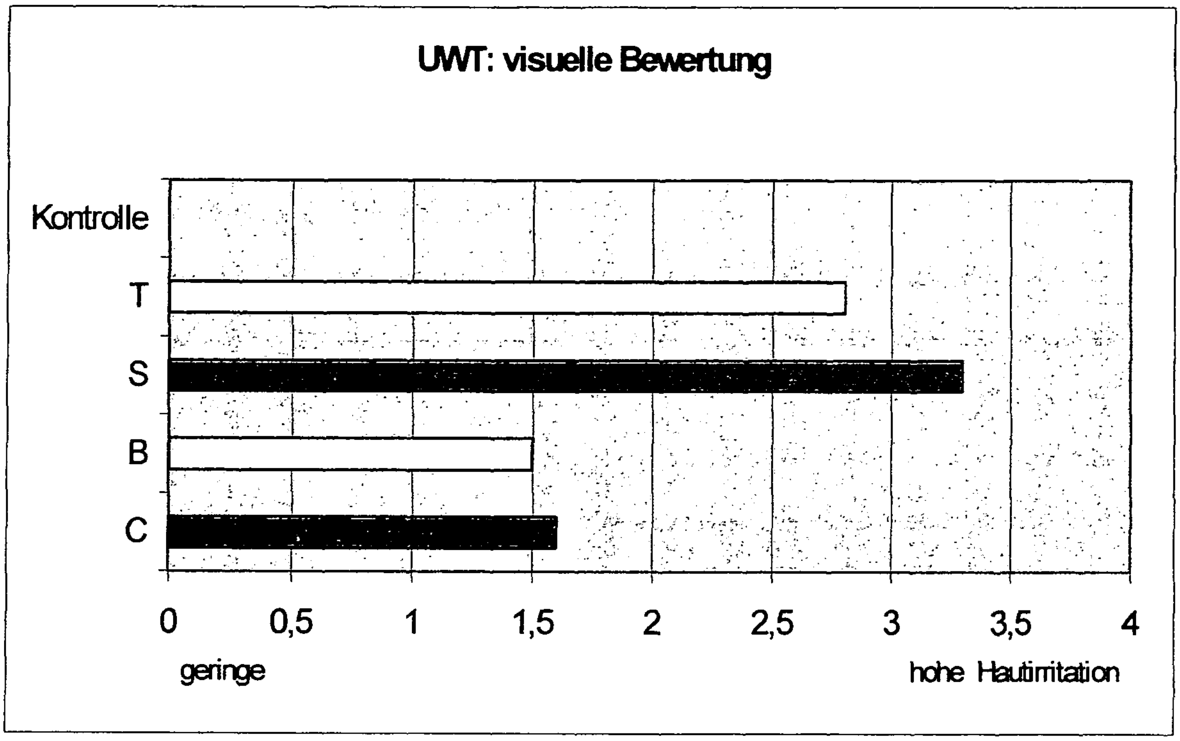
UWT: visuelle Bewertung
Kontrolle
T
S
B
C
0
0,5
1
1,5
2
2,5
3
3,5
4
geringe
hohe Hautirritation

Fig. 3

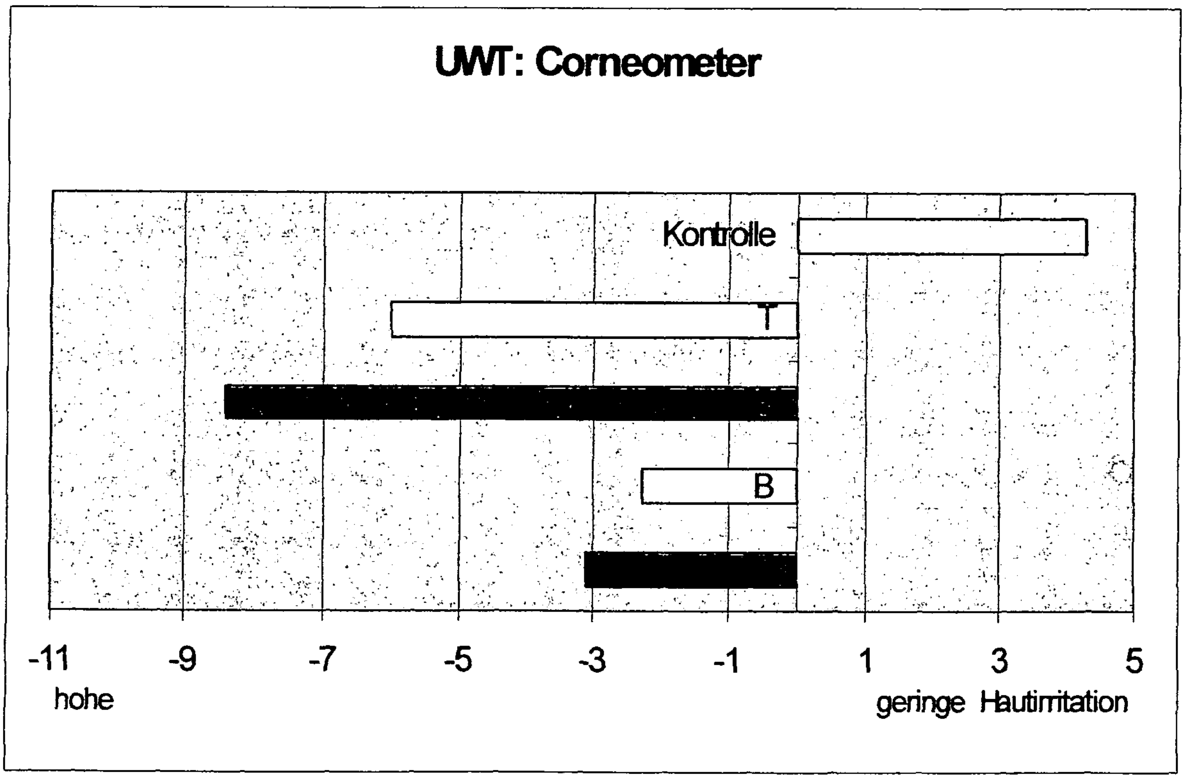
UWT: Corneometer
Kontrolle
T
B
-11
-9
-7
-5
-3
-1
1
3
5
hohe
geringe Hautirritation

Fig. 4

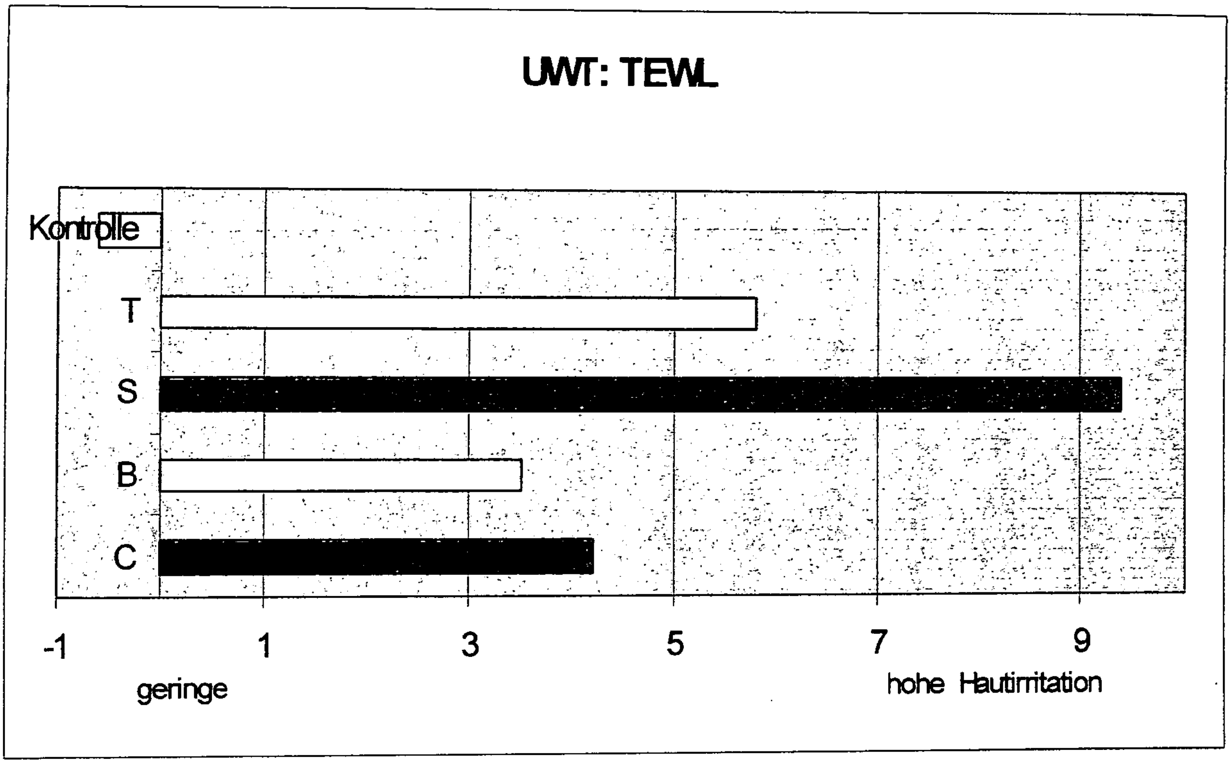
UWT: TEWL
Kontrolle
T
S
B
C
-1
1
3
5
7
9
geringe
hohe Hautirritation

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 4335933 A1
- WO 9906021 A
- DE 19748921 A1
- EP 1152051 A2
- DE 19748921 C2
- DE 4009534 A1
- DE 19916036 A1
- EP 1152051 A1
- WO 19916036 A1


### In der Beschreibung aufgeführte Nicht-Patentliteratur


- **PJ. FROSCH ; A.M. KLIGMAN.** *Contact Dermatities,* 1979, vol. 5, 73
- **J. KRESKEN ; W.S. WASSILEW.** *H+G4,* 1992, 334
- **A. KLOTZ.** *Am. J. Cont. Derm.,* 2001, vol. 12 (1), 52
- **A. KLOTZ ; M. VEEGER.** *Pharm. Ztg.,* 2000, vol. 145 (35), 47-51